(19) Europäisches Patentamt
European Patent Office
Office européen des brevets

(11) Veröffentlichungsnummer: **0 055 214**
**B1**

# (12) EUROPÄISCHE PATENTSCHRIFT

(45) Veröffentlichungstag der Patentschrift: **22.05.85**

(51) Int. Cl.⁴: **C 07 D 239/38,** C 07 D 239/60, C 09 B 62/20

(21) Anmeldenummer: **81810490.3**

(22) Anmeldetag: **11.12.81**

(54) **2-Alkyl-5-alkylsulfonyl-4,6-dihalogenpyrimidine und Verfahren zu deren Herstellung.**

(30) Priorität: **17.12.80 CH 9301/80**

(43) Veröffentlichungstag der Anmeldung: **30.06.82 Patentblatt 82/26**

(45) Bekanntmachung des Hinweises auf die Patenterteilung: **22.05.85 Patentblatt 85/21**

(84) Benannte Vertragsstaaten: **CH DE FR GB LI**

(56) Entgegenhaltungen: **DE - A - 2 819 837**

(73) Patentinhaber: **CIBA-GEIGY AG, Postfach, CH-4002 Basel (CH)**

(72) Erfinder: **Hoegerle, Karl, Dr., Reinacherstrasse 68, CH-4053 Basel (CH)**
Erfinder: **Ohnemus, Kurt, Römerfeldstrasse 17, CH-4125 Riehen (CH)**

**EP 0 055 214 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-Alkyl-5-alkylsulfonyl-4,6-dihalogenpyrimidine, die sich z.B. zur Herstellung von Reaktivfarbstoffen eignen, Verfahren zu deren Herstellung sowie die dabei erhältlichen neuen Zwischenprodukte.

Es ist bekannt, das 2-Alkylsulfonyl-4-halogen-6-alkylpyrimidine zur Herstellung von Reaktivfarbstoffen eingesetzt werden können. Dabei erfolgt die Bildung des Reaktivfarbstoffs über das Halogenatom in 4-Stellung, und die Alkylsulfonylgruppe in 2-Stellung wirkt bei der Bildung der kovalenten Bindung zwischen Farbstoff und Substrat als Abgangsgruppe (vgl. z.B. US-PS 3 853 840). Reaktivfarbstoffe mit einer Alkylgruppe in 6-Stellung und einer Alkylsulfonylgruppe in 2-Stellung des Pyrimidinringes eignen sich zum Färben und Bedrucken cellulosehaltiger Materialien. Diese Farbstoffe werden auf der Faser ausschliesslich durch die Alkylsulfonylgruppe als Abgangsgruppe fixiert. Es bestand daher die Aufgabe, neue Reaktivkomponenten zu finden, die nach Umsetzung mit einem Chromophor zu einem Reaktivfarbstoff eine applikatorisch grössere Anwendungsbreite ergeben.

Es wurden nun neue 2-Alkyl-5-alkylsulfonyl-4,6-dihalogenpyrimidine gefunden, die zur Herstellung von Reaktivfarbstoffen verwendet werden können, die sich sowohl für den Einsatz in Druck- als auch in Foulardverfahren eignen. Dabei dienen die Halogenatome zur Bildung der Farbstoff-Reaktivkomponenten-Bindung wie auch zur Fixierung des Reaktivfarbstoffes auf der Faser. Die 5ständige Alkylsulfonylgruppe aktiviert die Halogenatome, nimmt selbst jedoch an den Reaktionen nicht teil.

Gegenstand der Erfindung sind somit neue 2-Alkyl-5-alkylsulfonyl-4,6-dihalogenpyrimidine der Formel I

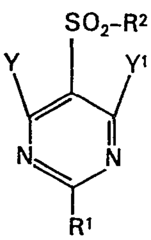

(I),

worin $R^1$ und $R^2$ unabhängig voneinander geradkettiges oder verzweigtes $C_{1-5}$-Alkyl und Y und $Y^1$ unabhänigig voneinander Chlor, Fluor oder Brom bedeuten.

Beispiele von Alkylgruppen $R^1$ oder $R^2$ sind: die Methyl-, Äthyl-, n-Propyl-, Isopropyl-, n-Butyl-, sek-Butyl-, tert-Butyl-, n-Pentyl-, 2- und 3-Pentylgruppe.

Bevorzugt sind Y und Y1 gleich und stellen Chlor, Fluor oder Brom dar. Besonders bevorzugt sind Verbindungen der Formel I, worin Y und $Y^1$ je Chlor oder Fluor, $R^1$ Methyl, Äthyl oder Isopropyl und $R^2$ Methyl oder Äthyl bedeuten. Ganz besonders bevorzugt sind Verbindungen der Formel I, worin Y und $Y^1$ je Chlor oder Fluor und $R^1$ und $R^2$ je Methyl bedeuten.

Als Beispiele spezifischer Verbindungen der Formel I seien erwähnt:

2-Methyl-5-methylsulfonyl-4,6-dichlor-, -4,6-dibrom- und -4,6-difluorpyrimidin,
2-Äthyl-5-methylsulfonyl-4,6-dichlor-und-4,6-difluorpyrimidin,
2-Methyl-5-äthylsulfonyl-4,6-dichlor- und -4,6-difluorpyrimidin,
2-n-Propyl-5-methylsulfonyl-4,6-dichlor- und -4,6-difluorpyrimidin,
2-Isopropyl-5-methylsulfonyl-4,6-dichlor-, -4,6-dibrom- und -4,6-difluorpyrimidin,
2-Methyl-5-methylsulfonyl-2-chlor-4-fluorpyrimidin,
2-n-Butyl-5-äthylsulfonyl-4,6-dichlor- und -4,6-difluorpyrimidin,
2-n-Pentyl-5-methylsulfonyl-4,6-dichlor- und -4,6-difluorpyrimidin,
2-Methyl-5-n-propylsulfonyl-4,6-dichlor- und -4,6-difluorpyrimidin,
2-Methyl-5-isopropylsulfonyl-4,6-dichlor- und -4,6-difluorpyrimidin,
2-Methyl-5-n-butylsulfonyl-4,6-dichlor- und -4,6-difluorpyrimidin,
2-Äthyl-2-äthylsulfonyl-4,6-dichlor- und -4,6-difluorpyrimidin,

Die Verbindungen der Formel I können z.B. dadurch hergestellt werden, dass man

a) ein Salz eines Dianions der Formel II

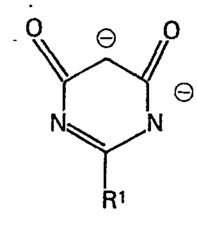

(II)

mit einer Verbindung der Formel III

$Z-SO_2-R^2$ (III)

zu einer Verbindung der Formel IV

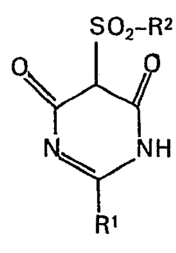

(IV)

oder einem Salz einer Verbindung der Formel IV umsetzt,

b) eine Verbindung der Formel IIa

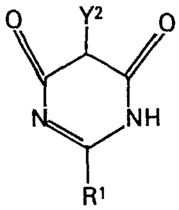

(IIa)

in Gegenwart eines polaren Lösungsmittels mit einer Verbindung der Formel IIIa

$Z^1-SO_2-R^2$ (IIIa)

zu einer Verbindung der Formel IV umsetzt und die gemäss a) oder b) erhaltene Verbindung der Formel IV oder deren Salz durch Behandlung mit einem Chlor, Brom und/oder Fluor einführenden Mittel zu einer Verbindung der Formel I umsetzt, oder

c) eine Verbindung der Formel IIb

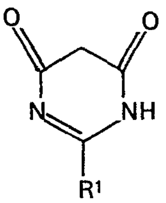

(IIb)

in Gegenwart eines Chlor, Brom und/oder Fluor einführenden und wasserabspaltenden Mittels mit einer Verbindung der Formel IIIb

$(R^2)_2SO$ (IIIb)

zu einer Verbindung der Formel V

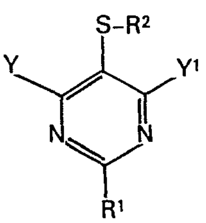

(V)

umsetzt, die Verbindung der Formel V mit einem milden Oxidationsmittel zu einer Verbindung der Formel I oxidiert und Verbindungen der Formel I, worin eines von Y und $Y^1$ Chlor und das andere Chlor, Brom oder Fluor bedeuten, gegebenenfalls einer Umhalogenierung unterwirft. In den obigen Formeln haben $R^1$, $R^2$, Y und $Y^1$ die unter Formel I angegebene Bedeutung,

$Y^2$ bedeutet Chlor, Fluor oder Brom, bevorzugt Chlor,

Z stellt ein Halogenatom, wie Chlor, Fluor oder Brom, bevorzugt Chlor, oder $-O-SO_2-R^2$ dar und

$Z^1$ ist ein Alkalimetall- oder Erdalkalimetallkation, bevorzugt das Natrium- oder Kaliumkation.

Vorzugsweise stellt man die Verbindungen der Formel I nach dem oben unter c) beschriebenen Verfahren her, d.h. durch Umsetzung einer Verbindung der Formel IIb in Gegenwart eines Chlor, Brom und/oder Fluor einführenden und wasserabspaltenden Mittels mit einer Verbindung der Formel IIIb und anschliessende Oxidation sowie gegebenenfalls Umhalogenierung.

Die Verbindungen der Formel II können in verschiedenen Grenzstrukturen vorliegen, während die Verbindungen der Formeln IIa, IIb und IV bzw. deren Salze in mehreren tautomeren Formen existieren können. Um die Beschreibung zu vereinfachen, sind diese Verbindungen in den Formeln nur in einer dieser Grenzstrukturen bzw. tautomeren Formen dargestellt. Es sei jedoch ausdrücklich betont, dass sich die Beschreibung, einschliesslich der Ansprüche, stets auf Verbindungen der genannten Art in irgendeiner ihrer Grenzstrukturen bzw. tautomeren Formen bezieht.

Die Verbindungen der Formel IV, deren Salze sowie die Verbindungen der Formel V sind neu und ebenfalls Gegenstand der Erfindung. Dabei haben $R^1$, $R^2$, Y und $Y^1$ mit Vorteil die oben angegebenen bevorzugten Bedeutungen.

Als Salze von Dianionen der Formel II bzw. Verbindungen der Formel IV kommen Salze mit anorganischen oder organischen Basen in Betracht, z.B. Alkalimetall- und Erdalkalmetallsalze sowie quaternäre Ammoniumsalze, wie Tetraalkylammonium- oder Benzyltrialkylammoniumsalze mit je 1–8 und bevorzugt je 1–4 C-Atomen in den Alkylgruppen. Beispiele derartiger Salze sind die Lithium-, Natrium-, Kalium- und Calciumsalze, Benzyltrimethylammonium-, Benzyltriäthylammonium-, Tetramethylammoniumsalze. Bevorzugt sind Tetraalkylammonium- und Benzyltrialkylammoniumsalze mit je 1–4 C-Atomen in den Alkylgruppen, besonders Tetraäthylammonium- und Benzyltriäthylammoniumsalze, und Alkalimetallsalze, besonders Natrium- und Kaliumsalze.

Die Herstellung der oben erwähnten Salze kann auf an sich bekannte Weise vor der weiteren Umsetzung oder im Reaktionsmedium selbst, d.h. in situ, erfolgen.

Die Ausgangsprodukte der Formeln II, IIa, IIb, III, IIIa und IIIb sind bekannt oder können nach an sich bekannten Methoden hergestellt werden.

Geeignete Ausgangsprodukte der Formeln III, IIIa und IIIb sind z.B.: Methansulfonsäurechlorid, Methansulfonsäurebromid, Äthansulfonsäurechlorid, Propansulfonsäurechlorid, Isopropan-, n-Butan-, sek-Butan-, n-Pentan-, 2- und 3-Pentansulfonsäurechlorid; Natrium-, Kalium- und Calciummethansulfinat, Natrium- und Kaliumäthansulfinat, -propansulfinat, -isopropansulfinat, -n-butansulfinat, -sek-butansulfinat, -tert-butansulfinat, -n-pentansulfinat, -2- und -3-pentansulfinat; Dimethylsulfoxid, Diäthylsulfoxid, Di-n-propylsulfoxid, Di-isopropylsulfoxid, Di-n-butylsulfoxid, Di-sek-butylsulfoxid, Di-n-pentylsulfoxid, Di-2-pentylsulfoxid und Di-3-pentylsulfoxid.

Mit Vorteil verwendet man Verbindungen der

Formel II, IIa, IIb, III, IIIa, oder IIIb bzw. Salze von Verbindungen der Formel II, worin $R^1$ und $R^2$ die oben angegebenen bevorzugten Bedeutungen haben, Z ein Chloratom ist und $Z^1$ das Natrium- oder Kaliumkation darstellt.

Die Umsetzung der Salze von Dianionen der Formel II mit den Verbindungen der Formel III wird zweckmässig in Gegenwart einer starken anorganischen oder organischen Base vorgenommen. Geeignete Basen sind z.B. Alkalimetall- oder Erdalkalimetallhydroxide, -amide und -hydride, sowie Alkyllithiumverbindungen, z.B. Methyllithium, n-Butyllithium, oder Lithiumdialkylamide, z.B. Lithiumdiiosopropylamid.

Die obige Umsetzung kann in wässrigem Medium, in einem inerten organischen Lösungsmittel oder einem Gemisch verschiedener inerter organischer Lösungsmittel, in einem Gemisch von Wasser und einem oder mehreren inerten organischen Lösungsmitteln oder auch in einem Lösungsmittel-Zweiphasensystem vorgenommen werden. Als organische Lösungsmittel werden vorzugsweise aprotische polare Lösungsmittel verwendet. Geeignete derartige Lösungsmittel sind beispielsweise N,N-Dialkylamide von aliphatischen Monocarbonsäuren mit 1-3 C-Atomen im Säureteil, wie N,N-Dimethylformamid, N,N-Dimethylacetamid und N,N-Diäthylacetamid; aliphatische und cycloaliphatische Ketone, wie Aceton, Methyläthylketon, Cyclopentanon und Cyclohexanon; cyclische Amide, wie N-Methyl-2-pyrrolidon, N-Acetyl-2-pyrrolidon, N-Methyl-ε-caprolactam; Dialkylsulfoxide, wie Dimethyl- und Diäthylsulfoxid; Pyridin.

Als Lösungsmittel-Zweiphasensysteme kommen besonders Systeme Wasser/organisches Lösungsmittel, wie Wasser/Methylenchlorid oder Wasser/Toluol, in Betracht.

Bevorzugt ist die Umsetzung in wässrigem Medium bei einem pH-Wert von 11,0 oder höher, vorzugsweise zwischen 11,5 und 12,5, wobei die Salze von Dianionen der Formel II im allgemeinen in situ gebildet werden. Als Basen verwendet man dabei vor allem Natrium- oder Kaliumhydroxid.

Für die Umsetzung in organischem Medium verwendet man als Base mit Vorteil nichtwässrige Basen, wie Alkyllithium, z.B. Butyllithium oder Alkalimetall-amide und -hydride, wie Lithiumdiisopropylamid und Natriumhydrid.

Die Umsetzung der Salze von Dianionen der Formel II mit den Verbindungen der Formel III wird zweckmässig bei Temperaturen zwischen -20°C und +100°C, vorzugsweise bei einer Temperatur zwischen +10 und +50°C und gegebenenfalls in Gegenwart eines Phasentransfer-Katalysators durchgeführt. Als Phasentransfer-Katalysatoren können z.B. Ammonium- oder Sulphoniumsalze, besonders Tetraalkyl- oder Benzyltrialkylammoniumhalogenide, vor allem -chloride, wie Benzyltriäthylammoniumchlorid, eingesetzt werden.

Nach beendeter Umsetzung fallen die Verbindungen der Formel IV direkt oder nach Ansäuern der Reaktionslösung, z.B. mit konzentrierter Salzsäure oder konzentrierter Schwefelsäure, in Form von Salzen, und zum Teil auch als freie Säuren aus. Diese können zur Reinigung aus Wasser umkristallisiert werden. Aus den Salzen können gewünschtenfalls auf an sich bekannte Weise die freien 5-Alkylsulfonylderivate gewonnen werden, z.B. indem man die Salze mit stark sauren Ionenaustauschern, wie $SO_3^-$-Gruppen enthaltenden Kunstharzen, behandelt. Andererseits ist es auch möglich, die Verbindungen der Formel IV in an sich bekannter Weise in Salze der vorerwähnten Art überzuführen oder die nach der Reaktion anfallenden Salze in andere Salze umzuwandeln.

Als polare Lösungsmittel für die Umsetzung von Verbindungen der Formel IIa mit den Alkalimetall- oder Erdalkalimetallsulfinaten der Formel IIIa können z.B. N,N-disubstituierte Amide von aliphatischen Monocarbonsäuren mit 1-3 C-Atomen im Säureteil, wie N,N-Dimethylformamid, N,N-Dimethylacetamid, N,N-Diäthylacetamid, N-Methyl-N-pehnylacetamid und N,N-Dimethylmethoxy-acetamid; cyclische Amide, wie N-Methyl-2-pyrrolidon, N-Acetyl-2-pyrrolidon und N-Methyl-ε-caprolactam; Tetraalkylharnstoffe, wie Tetramethylharnstoff, oder Dialkylsulfoxide, wie Dimethylsulfoxid und Diäthylsulfoxid, bzw. Gemische derartiger Lösungsmittel verwendet werden. Bevorzugte Lösungsmittel sind Dialkylsulfoxide, vor allem Dimethylsulfoxid oder Diäthylsulfoxid. Die Reaktionstemperaturen für diese Umsetzung liegen zweckmässig zwischen 50 und 200°C, bevorzugt 80 und 150°C.

Als Chlor, Brom und/oder Fluor einführende und gegebenenfalls wasserabspaltende Mittel für die Überführung von Verbindungen der Formel IV bzw. deren Salzen in Verbindungen der Formel I oder die Umsetzung von Verbindungen der Formel IIb mit Verbindungen der Formel IIIb kommen z.B. in Betracht: Phosphor(V)oxichlorid, -bromid und -fluorid; Methylphosphor(V)oxidichlorid ($CH_3POCl_2$), Phenylphosphor(V)oxidichlorid ($C_6H_5POCl_2$), Phosgen, Thionylbromid, Thionylchlorid und $SF_4$. Es können auch Gemische verschiedener derartiger Verbindungen, z.B. Gemische aus Phosphor(V)oxichlorid und Phosphor(V)oxibromid oder aber gegebenenfalls wasserabspaltende Halogenierungsmittel mit unterschiedlichen Halogenatomen, z.B. sogenannte gemischte Thionylhalogenide, wie SOClF oder SOBrCl, verwendet werden. Dabei erhält man Verbindungen der Formel I bzw. V, worin Y und $Y^1$ unterschiedliche Bedeutung haben. Bevorzugt verwendet man ein Chlor einführendes Mittel, vor allem Phosphor(V)oxichlorid.

Im allgemeinen ist es zweckmässig, die Überführung der Verbindungen der Formel IV bzw. von Salzen davon in Verbindungen der Formel I in Gegenwart eines Reaktionsbeschleunigers auszuführen. Geeignete Reaktionsbeschleuniger sind beispielsweise aliphatische oder aromatische tertiäre Basen, wie Triäthylamin, N,N-Dimethylanilin, N,N-Diäthylanilin, oder N,N-disubstituierte Amide von aliphatischen Carbonsäuren, wie N,N-Dimethylformamid und N,N-Dimethylacetamid. Diese Reaktionsbeschleuniger

werden im allgemeinen in einer Menge von 1-200 Mol%, bezogen auf die verwendete Menge der Verbindung der Formel IV oder deren Salz, eingesetzt. Vorzugsweise verwendet man äquimolare Mengen Reaktionsbeschleuniger.

Die vorerwähnten Umsetzungen mit einem Chlor, Brom und/oder Fluor einführenden und gegebenenfalls wasserabspaltenden Mittel gemäss Verfahrensvariante b) oder c) werden bevorzugt in einem inerten organischen Lösungsmittel vorgenommen. Geeignet hierfür sind z.B. aromatische Kohlenwasserstoffe, wie Xylole, Toluol, Chlorbenzol oder Nitrobenzol, sowie chlorierte aliphatische oder cycloaliphatische Kohlenwasserstoffe, wie Dichlormethan, Trichloräthylen, Tetrachlorkohlenstoff und Cyclohexylchlorid.

Als Lösungsmittel kann auch – vor allem für die Überführung von Verbindungen der Formel IV bzw. deren Salzen in Verbindungen der Formel I – überschüssiges Chlor, Brom und/oder Fluor einführendes Mittel, besonders Phosphor(V)oxichlorid, dienen.

Die Einführung von Chlor, Brom und/oder Fluor gemäss Verfahrensvariante b) wird zweckmässig bei einer Temperatur zwischen 20 und 250°C und vorzugsweise bei einer Temperatur zwischen 50 und 150°C vorgenommen.

Die Umsetzung von Verbindungen der Formel IIb in Gegenwart eines Chlor, Brom und/oder Fluor einführenden und gleichzeitig dehydratisierenden Mittels mit einer Verbindung der Formel IIIb wird vorzugsweise bei einer Temperatur zwischen 0 und 150°C, und in Gegenwart eines inerten organischen Lösungsmittels, vor allem eines chlorierten aliphatischen Kohlenwasserstoffs, wie Dichlormethan, durchgeführt.

Beispiele milder Oxidationsmittel für die Umsetzung der Verbindungen der Formel V zu Verbindungen der Formel I sind Peressigsäure, 3-Chlorperbenzoesäure, Perbenzoesäure, Wasserstoffperoxid, Natriumjodat, Perlaurinsäure, Jodbenzodichlorid, N-Chlorsuccinimid, N-Bromsuccinimid und Chlor. Die obige Umsetzung wird zweckmässig in Gegenwart eines inerten Lösungsmittels vorgenommen. Als Lösungsmittel kommen z.B. Dichlormethan, Chloroform, Essigsäure, Essigsäureanhydrid und Wasser oder deren Gemische in Frage. Die Oxidationstemperaturen liegen im allgemeinen zwischen -50°C und +50°C. Bevorzugte Oxidationsmittel sind Peressigsäure und 3-Chlorperbenzoesäure. Ähnliche Oxidationsreaktionen sind beispielsweise in Tetrahedron Letters, 1973, S. 2365 beschrieben; siehe dazu auch «Organic Compounds of Bivalent Sulfur», Band 2, Seite 4 (Chemical Publishing Co., New York, 1960).

Gemäss Verfahrensvariante a), b) und c) erhaltene Verbindungen der Formel I, worin eines von Y und $Y^1$ Chlor und das andere Chlor, Brom oder Fluor darstellen, können gewünschtenfalls bis zum Ersatz von einem oder beiden Chloratomen mit einem Bromierungs- oder Fluorierungsmittel, wie Phosphortribromid, wasserfreiem Fluorwasserstoff, Alkalimetallfluoriden oder Kaliumfluorosulfinat, umgesetzt werden. So können z.B. Verbindungen der Formel I, worin Y und $Y^1$ je Chlor bedeuten, in die Brom- oder Fluoranalogen übergeführt werden, indem man die genannten Verbindungen der Formel I durch Umsetzung mit Phosphortribromid, das auch als Lösungsmittel dienen kann, in das entsprechende 4,6-Dibrompyrimidinderivat überführt, oder indem man Verbindungen der Formel I mit Y und $Y^1$ = Chlor durch Umsetzung mit wasserfreiem Fluorwasserstoff, mit Kaliumfluorosulfinat oder einem Alkalimetallfluorid unverdünnt oder in Gegenwart eines hochsiedenden aprotischen organischen Lösungsmittels in die entsprechende 4,6-Difluorverbindung überführt. Geeignete Lösungsmittel für diese Umhalogenierungsreaktionen sind z.B. aromatische Kohlenwasserstoffe, wie Toluol und Xylole; N,N-Dialkylamide von aliphatischen Monocarbonsäuren der vorerwähnten Art, wie N,N-Dimethylformamid und N,N-Dimethylacetamid; cyclische Äther und cyclische Amide, wie Tetrahydrofuran, Tetrahydropyran, N-Methyl-2-pyrrolidon und N-Acetyl-2-pyrrolidon; Hexamethylphosphorsäuretriamid (Hexametapol); N,N,N',N'-Tetramethylharnstoff, Tetrahydrothiophendioxid (Sulfolan). Die Reaktionstemperaturen für die Umhalogenierung liegen zweckmässig zwischen 20 und 250°C, und bevorzugt zwischen 50 und 150°C.

Die Verbindungen der Formel I wie auch die Zwischenprodukte der Formel IV bzw. deren Salze und die Zwischenprodukte der Formel V lassen sich nach dem erfindungsgemässen Verfahren unter milden Reaktionsbedingungen in guten Ausbeuten herstellen.

Die Verbindungen der Formel I stellen – wie schon erwähnt – wertvolle Zwischenprodukte dar, die sich insbesondere zur Herstellung von Reaktivfarbstoffen eignen. Durch Kondensation von Verbindungen der Formel I, insbesondere Verbindungen der Formel I, worin $R^1$, $R^2$, Y und $Y^1$ die oben angegebenen bevorzugten Bedeutungen haben, mit aminogruppenhaltigen Farbstoffen erhält man faserreaktive Farbstoffe, die sich durch hohen Fixiergrad auszeichnen.

Sie eignen sich zum Färben und Bedrucken der verschiedensten Materialien, wie Seide, Leder, Wolle, Superpolyamidfasern und Superpolyurethanen, insbesondere aber cellulosehaltiger Materialien faseriger Struktur, wie Leinen, Zellstoff, regenerierte Cellulose und vor allem Baumwolle. Sie eignen sich zum Färben nach dem Foulardfärbeverfahren, wonach die Ware mit wässrigen und gegebenenfalls auch salzhaltigen Farbstofflösungen imprägniert wird, und die Farbstoffe nach einer Alkalibehandlung oder in Gegenwart von Alkali, gegebenenfalls unter Wärmeeinwirkung fixiert werden.

Sie eignen sich auch zum Druck, insbesondere auf Baumwolle, ebenso aber auch zum Bedrukken von stickstoffhaltigen Fasern, z.B. Wolle, Seide, oder Wolle enthaltenden Mischgeweben.

Beispiel 1

Herstellung von 2-Methyl-4,6-dihydroxy-5-methylsulfonylpyrimidin.

a) 12,6 g (0,1 Mol) 2-Methyl-4,6-dihydroxypyrimidin werden in 100 ml Wasser und 10 ml 10-N Natronlauge gelöst. Der pH-Wert beträgt 11,7. Dann werden 0,1 g Benzyltriäthylammoniumchlorid zugegeben, und innerhalb von ca. 3 Stunden werden 22,9 g (0,2 Mol) Methansulfochlorid eingetropft. Der pH-Wert wird mit 5-N Natronlauge bei 11,5–12 und die Temperatur durch Kühlung bei 25–30°C gehalten. Die Reaktionslösung wird noch zwei Stunden bei Raumtemperatur (20–25°C) nachgerührt, dann durch Zugabe von ca. 15 ml 10-N Salzsäure auf pH 5 gestellt. Der ausgefallene Niederschlag von unumgesetztem Ausgangsmaterial wird abfiltriert, und das Filtrat wird im Vakuum zur Trockene eingedampft. Das rohe, noch mit Ausgangsmaterial und Natriummethylsulfonat verunreinigte Produkt wird am Hochvakuum scharf getrocknet. Eine gereinigte analysenreine Probe der Verbindung schmelzt nicht bei 330°C, sondern zersetzt sich langsam, unter Verfärbung nach braun-schwarz ab ca. 280°C. Die Verbindung ist charakterisiert durch die Mikroanalyse sowie die IR- und $13_C$-Spektren:

Analyse:

Ber.: C 35,30; H 3,95; N 13,72; O 31,34; S 15,70%
Gef.: C 35,30; H 3,84; N 13,86; O 31,25; S 15,62%

Wichtigste IR-Banden:

1660 $cm^{-1}$, 1634 $cm^{-1}$, 1357 $cm^{-1}$, 1300 $cm^{-1}$, 1136 $cm^{-1}$, 962 $cm^{-1}$, 788 $cm^{-1}$,.

$13_C$-NMR Daten:

C-2 162,0 ppm, C-4 und C-6 158,3 ppm, C-5 99,1 ppm, $CH_3SO_2$ 44,0 ppm, $CH_3$ in 2-Stellung 17,8 ppm.

b) 16,0 g (0,1 Mol) 2-Methyl-5-chlor-4,6-dihydroxypyrimidin werden in 50 ml Dimethylsulfoxid mit 11,2 g (0,11 Mol) Natrium-methylsulfinat während 4 Stunden bei 125–130°C gerührt. 90°C warm klarfiltriert, und das Filtrat wird mit Chloroform versetzt. Der flockige Niederschlag wird abfiltriert und gut getrocknet. Man erhält 9,4 g einer ca. 90%igen Ware, die roh für die weitere Umsetzung eingesetzt wird.

Beispiel 2

Herstellung von 2-Methyl-4,6-dichlor-5-methylsulfonylpyrimidin.

a) 35 g trockenes rohes 2-Methyl-4,6-dihydroxy-5-methylsulfonylpyrimidin [nach Beispiel 1, Variante a) oder b) hergestellt] wird langsam zu einer Lösung von 12,1 h N,N-Dimethylanilin in 150 ml Phosphor(V)oxichlorid zugegeben. Die Suspension wird langsam erwärmt. Bei 80°C Innentemperatur findet die Reaktion unter kräftiger Salzsäureentwicklung statt. Die Suspension wird weiter bis zum Rückfluss erwärmt und 20 Stunden bei dieser Temperatur gehalten. Dann wird das Phosphor(V)oxichlorid abdestilliert, der Rückstand wird in Dichlormethan aufgenommen, die Lösung mit Eiswasser gewaschen und über Magnesiumsulfat getrocknet. Nach Abdestillieren des Dichlormethans wird durch Destillation bei 130°C Badtemperatur und 26 mbar 2-Methyl-4,6-dichlor-pyrimidin entfernt. Anschliessend wird das Produkt bei 140°C Badtemperatur und 0,9 mbar sublimiert. Kristallisation aus Tetrachlorkohlenstoff gibt reines 2-Methyl-4,6-dichlor-5-methylsulfonylpyrimidin vom Smp. 146–147°C.

Analyse:

Ber.: C 29,89; H 2,51; N 11,62; S 13,30; Cl 29,41%
Gef.: C 29,81; H 2,43; N 11,61; S 13,05; Cl 29,40%

b) 20,9 g (0,1 Mol) 2-Methyl-5-methylthio-4,6-dichlor-pyrimidin werden in einer Lösung von 10 ml Acetanhydrid in 150 ml Essigsäure gelöst. Unter gutem Rühren werden innerhalb einer Stunde 42 g 40%ige (0,22 Mol) Peressigsäure zugetropft. Die Temperatur wird durch leichte Kühlung auf 40–45°C gehalten. Die Reaktionslösung wird noch 4–5 Stunden bei dieser Temperatur gehalten, bei Raumtemperatur über Nacht ausgerührt und dann auf 15°C abgekühlt. Der Niederschag wird filtriert und aus Petroläther umkristallisiert. Das Produkt hat den Smp. 138–40°C. Das IR-Spektrum der so erhaltenen Verbindung ist identisch mit demjenigen der gemäss 2a) erhaltenen Substanz.

Das im obigen Beispiel b) verwendete Ausgangsmaterial wird wie folgt hergestellt:

37,8 g (0,3 Mol) 2-Methyl-4,6-dihydroxypyrimidin werden in 150 ml Phosphor(V)oxichlorid und 100 ml Dichlormethan suspendiert. Innerhalb von 2½ Stunden werden unter Kühlung bei 20–25°C 26,6 g (0,34 Mol Dimethylsulfoxid zugetropft. Das Reaktionsgemisch wird über Nacht bei Raumtemperatur gerührt und dann langsam erwärmt. Bei 50–55°C setzt Chlorwasserstoffentwicklung ein. Das Dichlormethan wird dabei abdestilliert, und die Lösung wird während 12 Stunden am Rückfluss gekocht. Dann wird der Kolbeninhalt auf Wasser gegossen und die Temperatur durch Eiszugabe auf ca. 25°C gehalten. Das Produkt wird mit Dichlormethan extrahiert, die Lösung über Magnesiumsulfat getrocknet, eingedampft, und der Rückstand wird im Hochvakuum destilliert. Das Destillat vom Sdp. 50–64°C/0,13 mbar gibt aus Petroläther farblose Kristalle vom Smp. 59–60°C.

Analyse:

Ber.: C 34,46; H 2,89; N 13,40; S 15,33; Cl 33,91%
Gef.: C 34,52; H 2,82; N 13,49; S 14,96; Cl 34,26%

Beispiel 3

2-Methyl-5-methylsulfonyl-4,6-difluorpyrimidin.

Einer Lösung von 14,5 g (0,06 Mol) 2-Methyl-5-methylsulfonyl-4,6-dichlorpyrimidin in 400 ml absolutem Toluol werden 82 g (0,55 Mol) Kaliumfluorosulfinat zugegeben. Die Suspension wird langsam bis zum Rückfluss erwärmt und 18 Stunden beim Sieden gehalten. Dann wird über Kieselgur filtriert, und der Filterrückstand wird mehrmals mit trockenem Toluol gewaschen. Die vereinigten Filtrate werden bei 25–30°C am Rotationsverdampfer eingeengt, und der feste Eindampfrückstand von 8,8 g wird durch fraktionierte Sublimation bei 50–60°C am Wasserstrahlvakuum gereinigt. Die Reinsubstanz zeigt den Smp. 120–23°C.

Beispiel 4

Herstellung von 2-Isopropyl-4,6-dichlor-5-äthylsulfonylpyrimidin.

Zu einer Suspension von 15,4 g (0,1 Mol) 2-Isopropyl-4,6-dihydroxypyrimidin in 70 ml Essigsäure und 21 ml Acetanhydrid werden 12,3 g (0,116 Mol) Diäthylsulfoxid zugesetzt. Die Suspension wird innerhalb von zwei Stunden auf 95° erwärmt und sechs Stunden bei 95° nachgerührt. Die klare Lösung wird kalt mit 150 ml Eiswasser versetzt und die gelbe Fällung abfiltriert und mit wenig Wasser gewaschen.

Filtrat und Waschwasser werden zur Trockene eingedampft und der Rückstand zusammen mit dem Niederschlag am Hochvakuum bei 60° getrocknet. Die getrocknete Ware (21,9 g) wird mit 150 ml Phosphoroxichlorid und 12,6 ml Dimethylanilin zum Sieden erhitzt und 20 Stunden bei Siedetemperatur gehalten. Die erkaltete Lösung wird auf Wasser gegossen und durch Eiszugabe bei ca. 25° gehalten. Das sich abscheidende dunkle Öl wird in Methylenchlorid aufgenommen, über Magnesiumsulfat getrocknet, filtriert und eingedampft. Der Eindampfrückstand wird am Hochvakuum fraktioniert. Mit Sdp. 82–87°/0,13 mbar werden 10 g 2-Isopropyl-4,6-dichlor-5-äthylthiopyrimidin erhalten, die noch durch etwas 2-Isopropyl-4,6-dichlor-pyrimidin verunreinigt sind.

Analyse:
Ber.: C 43,04; H 4,82; N 11,15; S 12,76; Cl 28,23%
Gef.: C 42,95; H 4,62; N 11,18; S 11,06; Cl 29,72%

9,8 g (0,039 Mol) 2-Isopropyl-4,6-dichlor-5-äthylthiopyrimidin werden in 50 ml Essigsäure gelöst und dazu langsam 15,2 g 40%-ige Peressigsäure (0,08 Mol) zugetropft. Die exotherme Reaktion wird durch leichtes Kühlen bei 40° abgefangen. Nach 24-stündigem Rühren bei Raumtemperatur wird die klare Lösung mit dem 4-fachen Volumen Wasser verdünnt, der ausgefallene Niederschlag filtriert und in Methylenchlorid aufgenommen. Das wässrige Filtrat wird mit Methylenchlorid extrahiert und der Extrakt mit Natriumbicarbonatlösung neutral gewaschen. Die vereinigten Methylenchloridphasen werden über Magnesiumsulfat getrocknet, filtriert und zum Öl eingedampft. Der Rückstand (6,3 g) wird im Kugelrohrofen am Hochvakuum destilliert. Bei einer Ofentemperatur von $150°/3 \cdot 10^{-2}$ mbar destillieren 5,4 g 2-Isopropyl-4,6, 6-dichlor-5-äthylsulfonylpyrimidin als farbloses Öl.

Analyse:
Ber.: C 38,18; H 4,27; N 9,89; S 11,32%
Gef.: C 37,85; H 4,10; N 9,92; S 10,91%.

Beispiel 5

2-Methyl-4,6-dibrom-5-methylsulfonyl-pyrimidin

8,2 g 2-Methyl-4,6-dihydroxy-5-methylsulfonyl-pyrimidin werden mit 100 g Phosphoroxitribromid 9 Stunden bei 105–110° gerührt. Die erkaltete Reaktionsmasse wird in kleinen Portionen auf Wasser gegossen und die Temperatur durch Zugabe von Eis auf 20–25° gehalten. Anschliessend wird die wässrige Phase mit Dichlormethan extrahiert, die Extrakte über Magnesiumsulfat getrocknet und das Lösungsmittel abdestilliert. Der Rückstand wird im Hochvakuum sublimiert: Smp. 161–162°.

Beispiel 6

8,76 Teile des Farbstoffes der Formel

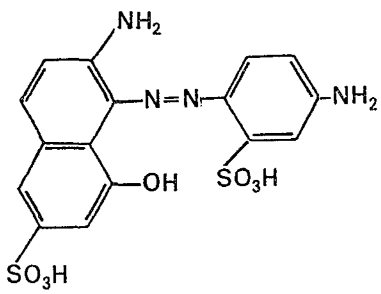

werden in 300 Teilen Wasser neutral gelöst und mit 6,68 Teilen Natriumacetat versetzt. Zur erhaltenen Lösung gibt man bei Zimmertemperatur unter kräftigem Rühren eine Lösung von 5,3 Teilen 2-Methyl-4,6-dichlor-5-methylsulfonylpyrimidin in 40 Teilen Aceton. Nach vollständiger Acylierung wird die Lösung klärfiltriert. Der entstandene Reaktivfarbstoff wird mit Kochsalz ausgesalzen, abfiltriert, mit einer gesättigten Lösung von Natriumdihydrogenphosphat gepuffert und im Vakuum bei ca. 50°C getrocknet.

Der so erhaltene Farbstoff färbt Baumwolle nach dem Pad-Steam-Verfahren in blaustichig roten Tönen.

**Patentansprüche**

1. Verbindungen der Formel I

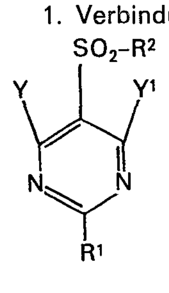

(I),

worin $R^1$ und $R^2$ unabhängig voneinander geradkettiges oder verzweigtes $C_{1-5}$-Alkyl und Y und $Y^1$ unabhängig voneinander Chlor, Fluor oder Brom bedeuten.

2. Verbindungen der Formel I nach Anspruch 1, worin Y und $Y^1$ gleich sind und Chlor, Fluor oder Brom bedeuten.

3. Verbindungen der Formel I nach Anspruch 1, worin Y und $Y^1$ je Chlor oder Fluor, $R^1$ Methyl, Äthyl oder Isopropyl und $R^2$ Methyl oder Äthyl bedeuten.

4. Verbindungen der Formel I nach Anspruch 1, worin Y und $Y^1$ je Chlor oder Fluor und $R^1$ und $R^2$ je Methyl bedeuten.

5. Verfahren zur Herstellung von Verbindungen der Formel I nach Anspruch 1, dadurch gekennzeichnet, dass man

a) ein Salz eines Dianions der Formel II

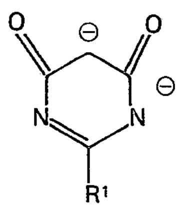

(II)

mit einer Verbindung der Formel III

$Z-SO_2-R^2$ (III)

zur einer Verbindung der Formel IV

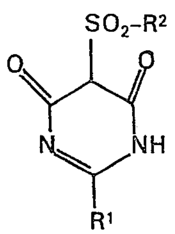

(IV)

oder einem Salz einer Verbindung der Formel IV umsetzt,

b) eine Verbindung der Formel IIa

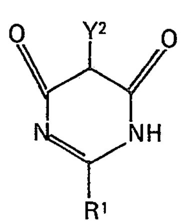

(IIa)

in Gegenwart eines polaren Lösungsmittels mit einer Verbindung der Formel IIIa

$Z^1-SO_2-R^2$ (IIIa)

zu einer Verbindung der Formel IV umsetzt und die gemäss a) oder b) erhaltene Verbindung der Formel IV oder deren Salz durch Behandlung mit einem Chlor, Brom und/oder Fluor einführenden Mittel zu einer Verbindung der Formel I umsetzt, oder

c) eine Verbindung der Formel IIb

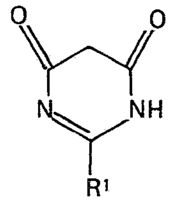

(IIb)

in Gegenwart eines Chlor, Brom und/oder Fluor einführenden und wasserabspaltenden Mittels mit einer Verbindung der Formel IIIb

$(R^2)_2SO$ (IIIb)

zu einer Verbindung der Formel V

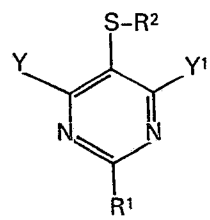

(V)

umsetzt, die Verbindung der Formel V mit einem milden Oxidationsmittel zu einer Verbindung der Formel I oxidiert und Verbindungen der Formel I, worin eines von Y und $Y^1$ Chlor und das andere Chlor, Brom oder Fluor bedeuten, gegebenenfalls einer Umhalogenierung unterwirft, wobei in den obigen Formeln $R^1$, $R^2$, Y und $Y^1$ die im Anspruch 1 angegebene Bedeutungen haben,

$Y^2$ Chlor, Fluor oder Brom bedeutet,

Z ein Halogenatom oder $-O-SO_2-R^2$ darstellt und

$Z^1$ ein Alkalimetall- oder Erdalkalimetallkation bedeutet.

6. Verfahren nach Anspruch 5, dadurch gekennzeichnet, dass man eine Verbindung der Formel IIb in Gegenwart eines Chlor, Brom und/oder Fluor einführenden und wasserabspaltenden Mittels mit einer Verbindung der Formel IIIb zu einer Verbindung der Formel V umsetzt, anschliessend zu einer Verbindung der Formel I oxidiert und gegebenenfalls umhalogeniert.

7. Verfahren nach Anspruch 5, dadurch gekennzeichnet, dass man die Umsetzung eines Salzes eines Dianions der Formel II mit einer Verbindung der Formel III in Gegenwart einer starken anorganischen oder organischen Base bei einer Temperatur zwischen -20°C und +100°C durchführt.

8. Verfahren nach Anspruch 7, dadurch gekennzeichnet, dass man die Umsetzung in wässrigem Medium bei einem pH-Wert von 11 oder höher vornimmt.

9. Verfahren nach Anspruch 7 oder 8, dadurch gekennzeichnet, dass man als Base Natrium- oder Kaliumhydroxid verwendet.

10. Verfahren nach Anspruch 5, dadurch gekennzeichnet, dass man als polares Lösungsmittel für die Umsetzung einer Verbindung der Formel IIa mit einer Verbindung der Formel IIIa ein Dialkylsulfoxid verwendet.

11. Verfahren nach Anspruch 10, dadurch gekennzeichnet, dass man Dimethylsulfoxid oder Diäthylsulfoxid verwendet.

12. Verfahren nach Anspruch 5, dadurch gekennzeichnet, dass man eine Verbindung der Formel IIIa verwendet, worin $Z^1$ das Natrium- oder Kaliumkation darstellt, und die Umsetzung bei einer Temperatur zwischen 50 und 200°C vornimmt.

13. Verfahren nach Anspruch 6, dadurch gekennzeichnet, dass man für die Umsetzung von Verbindungen der Formel V zu Verbindungen der Formel I Peressigsäure oder 3-Chlorperbenzoesäure als Oxidationsmittel verwendet und die Oxidation bei einer Temperatur zwischen -50°C und +50°C vornimmt.

14. Verbindungen der Formel IV

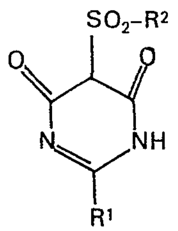
(IV)

oder deren Salze, worin $R^1$ und $R^2$ unabhängig voneinander geradkettiges oder verzweigtes $C_{1-5}$-Alkyl bedeuten.

15. Verbindungen der Formel V

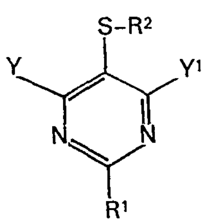
(V)

worin $R^1$ und $R^2$ unabhängig voneinander geradkettiges oder verzweigtes $C_1$-$C_5$-Alkyl und Y und $Y^1$ unabhängig voneinander Chlor, Fluor oder Brom bedeuten.

**Claims**

1. A compound of the formula I

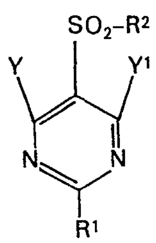
(I),

wherein each of $R^1$ and $R^2$ independently is straight chain or branched $C_1$-$C_5$ alkyl, and each of Y and $Y^1$ independently is chlorine, fluorine or bromine.

2. A compound of the formula I according to claim 1, wherein Y and $Y^1$ are the same and are chlorine, fluorine or bromine.

3. A compound of the formula I according to claim 1, wherein each of Y and $Y^1$ is chlorine or fluorine, $R^1$ is methyl, ethyl or isopropyl, and $R^2$ is methyl or ethyl.

4. A compound of the formula I according to claim 1, wherein each of Y and $Y^1$ is chlorine or fluorine and each of $R^1$ and $R^2$ is methyl.

5. A process for the preparation of a compound of the formula I according to claim 1, which process comprises

a) reacting a salt of a dianion of the formula II

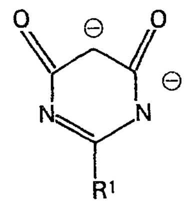
(II)

with a compound of the formula III

$Z$-$SO_2$-$R^2$ (III)

to a compound of the formula IV

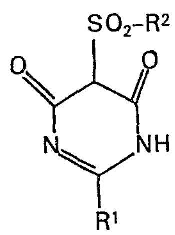
(IV)

or to a salt thereof; or

b) reacting a compound of the formula IIa

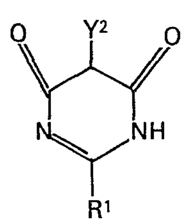
(IIa)

in the presence of a polar solvent, with a compound of the formula IIIa

$Z^1$-$SO_2$-$R^2$ (IIIa)

to a compound of the formula IV, and reacting the compound of the formula IV obtained in accordance with a) or b), or the salt thereof, by treatment with a chlorinating, brominating

and/or fluorinating agent, to a compound of the formula I; or

c) reacting a compound of the formula IIb

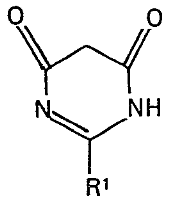

(IIb)

in the presence of a chlorinating, brominating and/or fluorinating and dehydrating agent, with a compound of the formula IIIb

$(R^2)_2SO$ (IIIb)

to a compound of the formula V

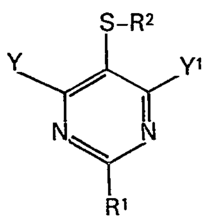

(V)

oxidising said compound of the formula V with a mild oxidising agent to a compound of the formula I and, if desired, subjecting the compound of the formula I, wherein one of Y and $Y^1$ is chlorine and the other is chlorine, bromine or fluorine, to transhalogenation, in which formulae above $R^1$, $R^2$, Y and $Y^1$ are as defined in claim 1, $Y^2$ is chlorine, fluorine or bromine, Z is a halogen atom or $-O-SO_2-R^2$, and $Z^1$ is the cation of an alkali metal or alkaline earth metal.

6. A process according to claim 5, which process comprises reacting a compound of the formula IIb, in the presence of a chlorinating, brominating and/or fluorinating and dehydrating agent, with a compound of the formula IIIb to give a compound of the formula V, then oxidising said compound of the formula V to a compound of the formula I and, if desired, transhalogenating the compound of the formula I.

7. A process according to claim 5, wherein the reaction of a salt of a dianion of the formula II with a compound of the formula III is carried out in the presence of a strong inorganic or organic base in the temperature range from -20 to +100°C.

8. A process according to claim 7, wherein the reaction is carried out in aqueous medium at or above a pH value of 11.

9. A process according to either of claims 7 or 8, wherein the base is sodium or potassium hydroxide.

10. A process according to claim 5, wherein the polar solvent for the reaction of a compound of the formula IIa with a compound of the formula IIIa is a dialkyl sulfoxide.

11. A process according to claim 10, which comprises the use of dimethyl sulfoxide or diethyl sulfoxide.

12. A process according to claim 5, which comprises the use of a compound of the formula IIIa in which $Z^1$ is the sodium or potassium cation, and in which process the reaction is carried out in the temperature range from 50 to 200°C.

13. A process according to claim 6, wherein the oxidising agent for the reaction of a compound of the formula V to a compound of the formula I is peracetic acid or 3-chloroperbenzoic acid, and the oxidation is carried out in the temperature range from -50 to +50°C.

14. A compound of the formula IV

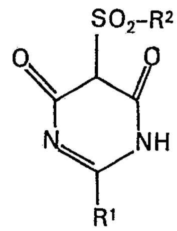

(IV)

wherein each of $R^1$ and $R^2$ independently is straight chain or branched $C_1$-$C_5$ alkyl, or a salt thereof.

15. A compound of the formula V

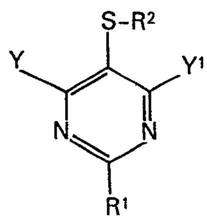

(V)

wherein each of $R^1$ and $R^2$ independently is straight chain or branched $C_1$-$C_5$ alkyl, and each of Y and $Y^1$ independently is chlorine, fluorine or bromine.

**Revendications**

1. Composés de formule I:

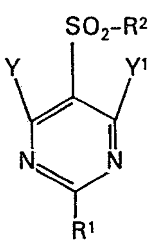

(I),

dans laquelle $R^1$ et $R^2$, indépendamment l'un de l'autre, sont des groupes alkyle en $C_{1-5}$ linéaires ou ramifiés, et Y et $Y^1$, indépendamment l'un de l'autre, sont des atomes de chlore, de fluor ou de brome.

2. Composés de formule I selon la revendication 1, dans lesquels Y et $Y^1$ sont identiques et représentent des atomes de chlore, de fluor ou de brome.

3. Composés de formule I selon la revendication 1, dans lesquels Y et $Y^1$ sont chacun un atome de chlore ou de fluor; $R^1$ est un groupe méthyle, éthyle ou isopropyle, et $R^2$ est un groupe méthyle ou éthyle.

4. Composés de formule I selon la revendication 1, dans lesquels Y et $Y^1$ sont chacun un atome de chlore ou de fluor, et $R^1$ et $R^2$ sont chacun un groupe méthyle.

5. Procédé pour la préparation des composés de formule I selon la revendication 1, caractérisé par le fait:

a) qu'on fait réagir un sel d'un dianion de formule II

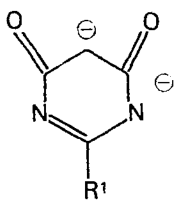

(II)

avec un composé de formule III:

$Z\text{-}SO_2\text{-}R^2$ (III)

pour avoir un composé de formule IV

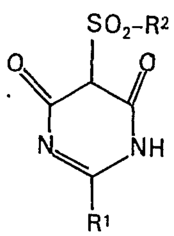

(IV)

ou un sel d'un composé de formule IV,

b) qu'on fait réagir un composé de formule IIa

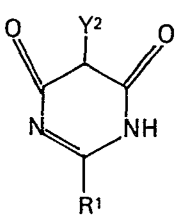

(IIa)

en présence d'un solvant polaire, avec un composé de formule IIIa

$Z^1\text{-}SO_2\text{-}R^2$ (IIIa)

pour avoir un composé de formule IV et qu'on transforme le composé de formule IV obtenu selon a) ou b) ou son sel par traitement avec un agent introduisant le chlore, le brome et/ou le fluor en un composé de formule I, ou bien

c) qu'on fait réagir un composé de formule IIb

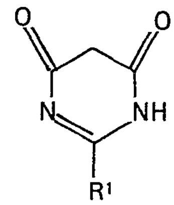

(IIb)

en présence d'un agent introduisant le chlore, le brome et/ou le fluor et déshydratant, avec un composé de formule IIIb

$(R^2)_2SO$ (IIIb)

pour avoir un composé de formule V

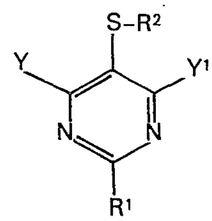

(V)

qu'on oxyde le composé de formule V avec un oxydant doux pour avoir un composé de formule I, et qu'on soumet les composés de formule I, dans lesquels un des Y et $Y^1$ est un atome de chlore et l'autre est un atome de chlore, de brome ou de fluor, éventuellement à une transhalogénation, $R^1$, $R^2$, Y et $Y^1$ dans les formules ci-dessus ayant les significations données dans la revendication 1,

$Y^2$ désignant un atome de chlore, de fluor ou de brome,

Z représentant un atome d'halogène ou le groupe $-O-SO_2-R^2$, et

$Z^1$ désignant un cation de métal alcalin ou de métal alcalino-terreux.

6. Procédé selon la revendication 5, caractérisé par le fait qu'on fait réagir un composé de formule IIb, en présence d'un agent introduisant le chlore, le brome et/ou le fluor et déshydratant, avec un composé de formule IIIb pour avoir un composé de formule V qu'on soumet à une oxydation et éventuellement à une transhalogénation pour avoir un composé de formule I.

7. Procédé selon la revendication 5, caractérisé par le fait qu'on effectue la réaction d'un sel d'un dianion de formule II avec un composé de formule III en présence d'une base minérale ou organique forte à une température comprise entre -20°C et +100°C.

8. Procédé selon la revendication 7, caractérisé par le fait qu'on effectue la réaction en milieu aqueux à un pH de 11 ou supérieur.

9. Procédé selon la revendication 7 ou 8, caractérisé par le fait qu'on utilise comme base l'hydroxyde de sodium ou l'hydroxyde de potassium.

10. Procédé selon la revendication 5, caractérisé par le fait qu'on utilise comme solvant polaire pour la réaction d'un composé de formule IIa avec un composé de formule IIIa un dialkylsulfoxyde.

11. Procédé selon la revendication 10, caractérisé par le fait qu'on utilise le diméthylsulfoxyde ou le diéthylsulfoxyde.

12. Procédé selon la revendication 5, caractérisé par le fait qu'on utilise un composé de formule IIIa dans lequel $Z^1$ représente un cation sodium ou un cation potassium, et qu'on effectue la réaction à une température comprise entre 50 et 200°C.

13. Procédé selon la revendication 6, caractérisé par le fait que pour la transformation du composé de formule V en composé de formule I on utilise comme oxydant l'acide peracétique ou l'acide 3-chloroperbenzoïque et que l'oxydation est effectuée à une température comprise entre -50°C et +50°C.

14. Composés de formule IV:

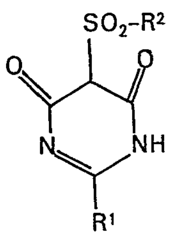

(IV)

ou leurs sels dans lesquels $R^1$ et $R^2$, indépendamment l'un de l'autre, désignent des groupes alkyle en $C_{1-5}$ linéaires ou ramifiés.

15. Composés de formule V:

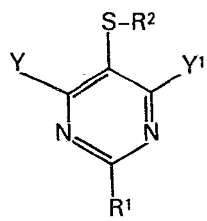

(V)

dans lesquels $R^1$ et $R^2$, indépendamment l'un de l'autre, représentent des groupes alkyle en $C_1$–$C_5$ linéaires ou ramifiés, et Y et $Y^1$, indépendamment l'un de l'autre, représentent des atomes de chlore, de fluor ou de brome.